# EUROPEAN PATENT APPLICATION

(11) **EP 3 260 038 A1**
(43) Date of publication of application: **27.12.2017**
(21) Application number: 16752235.8
(22) Date of filing: 28.01.2016
(51) Int. Cl.: A61B 1/313, A61B 17/34

(54) **ENDOSCOPE SYSTEM, ROTATION SENSOR AND TROCAR**

(30) Priority: 19.02.2015 JP 2015030626
(71) Applicant: Olympus Corporation, Hachioji-shi Tokyo 192-8507 (JP)
(72) Inventor: HARAGUCHI, Masafumi, Tokyo 192-8507 (JP); INOUE, Shintaro, Tokyo 192-8507 (JP)
(74) Representative: Schicker, Silvia
(86) International application number: PCT/JP2016/052475
(87) International publication number: WO 2016/132846

(57) **Abstract**

In order to detect the direction of rotation with high accuracy even when an existing endoscope is used irrespective of the orientation of the endoscope, an endoscope system (1) is provided with: an endoscope (6) that has an image-acquisition optical system at a distal end of an elongated insertion portion (5); a trocar (3) that is attached to a body surface (A) of a patient so as to penetrate the body surface (A) and that has a through-hole (2) though which the insertion portion (5) is made to pass; a rotation sensor (4) that is provided on the trocar (3) and that detects an amount of rotation of the insertion portion (5) about a longitudinal axis, the insertion portion (5) being located in the through-hole (2) in a passing-through state; a sign that is disposed at a position where it can be imaged by the image-acquisition optical system of the endoscope (6) when the insertion portion (5) is inserted into the trocar (3) and with which the position in a circumferential direction of the through-hole (2) can be identified; and an initial-angle detecting unit (7) that detects a relative angle between the insertion portion (5) and the through-hole (2) at the time of insertion on the basis of the sign in an image acquired by the image-acquisition optical system.

## Description

### {Technical Field}

The present invention relates to an endoscope system, a rotation sensor, and a trocar.

### {Background Art}

There is a known endoscope in which the direction of rotation of an endoscope body, when inserted into the body, about the axis is detected by using gravity and in which control is performed such that an image on a monitor is not rotated in response to the rotation of the endoscope body (for example, see PTL 1).

### {Citation List}

### {Patent Literature}

{PTL 1} Publication of Japanese Patent No. 3504681

### {Summary of Invention}

### {Technical Problem}

In the endoscope of PTL 1, because a sensor for detecting gravity needs to be provided on the endoscope itself, there is a disadvantage that the direction of rotation cannot be detected by using existing endoscopes. When the endoscope is used in a state in which the axis of the endoscope body is located nearly in the vertical direction, it is difficult to detect the direction of gravity, which may reduce the accuracy of detecting the direction of rotation of the endoscope body about the axis.

The present invention has been made in view of the above-described circumstances and provides an endoscope system, a rotation sensor, and a trocar with which it is possible to detect the direction of rotation with high accuracy, even when an existing endoscope is used, irrespective of the orientation of the endoscope.

### {Solution to Problem}

According to one aspect, the present invention provides an endoscope system including: an endoscope in which an image-acquisition optical system is provided at a distal end of an elongated insertion portion; a trocar that is attached to a body surface of a patient so as to penetrate the body surface and that has a through-hole through which the insertion portion is made to pass; a rotation sensor that is provided on the trocar and that detects an amount of rotation of the insertion portion about a longitudinal axis, the insertion portion being located in the through-hole in a passing-through state; a sign that is disposed at a position where it can be imaged by the image-acquisition optical system of the endoscope when the insertion portion is inserted into the trocar and with which a position in a circumferential direction of the through-hole can be identified; and an initial-angle detecting unit that detects a relative angle between the insertion portion and the through-hole at the time of insertion on the basis of the sign shown in an image acquired by the image-acquisition optical system.

According to this aspect, when the trocar is disposed in a hole opened in a body surface of a patient, and the insertion portion of the endoscope is inserted into the through-hole provided in the trocar from the distal end thereof, the rotation sensor provided on the trocar detects the amount of rotation of the insertion portion about the longitudinal axis. Furthermore, the image-acquisition optical system, which is provided at the distal end of the insertion portion, is actuated when the insertion portion is inserted into the trocar, thereby acquiring an image including the sign, with which the position in the circumferential direction of the through-hole can be identified, and detecting, by using the initial-angle detecting unit, the relative angle between the insertion portion and the through-hole at the time of insertion on the basis of the sign in the acquired image. Specifically, the absolute position of the insertion portion of the endoscope about the longitudinal axis can be detected on the basis of the relative angle at the time of insertion detected by the initial-angle detecting unit and the amount of rotation of the insertion portion detected by the rotation sensor after insertion.

In the above-described aspect, the sign may be disposed at a position where it is imaged after the amount of rotation of the insertion portion can be detected by the rotation sensor.

By doing so, when the insertion portion is inserted into the through-hole of the trocar, first, the rotation sensor starts detecting the amount of rotation of the insertion portion, and, next, the relative angle between the insertion portion and the through-hole is detected through acquisition of an image including the sign portion. Accordingly, at a time point when the amount of rotation of the insertion portion detected by the rotation sensor reaches a certain value, the relative angle between the insertion portion and the through-hole is detected, and, at that time point, the absolute position of the insertion portion with respect to the through-hole about the longitudinal axis can be detected. In a case in which the relative angle is first detected through image acquisition of the sign, and then the rotation sensor starts detecting the amount of rotation, there is a blank in the period from detection of the relative angle to detection of the amount of rotation, which makes it difficult to detect an accurate absolute position, in some cases.

In the above-described aspect, the sign may be provided at one place in the circumferential direction.

By doing so, as long as the sign is included in the image acquired by the image-acquisition optical system, the relative angle between the insertion portion and the through-hole about the longitudinal axis can be detected with high accuracy. In particular, in the case in which a direct-view endoscope is used and in which it is possible to acquire an image of the entirety of the through-hole at one time, detection of the relative angle is easy.

In the above-described aspect, the sign may be provided along a direction in which the insertion portion is inserted into the through-hole.

By doing so, it is possible to keep continuously detecting the sign during the process of inserting the insertion portion into the through-hole. Accordingly, it is possible to continuously detect and keep updating the relative angle between the insertion portion and the through-hole at the time of insertion.

In the above-described aspect, the sign may be a light-emitting portion that can emit light radially inward.

By doing so, even when illumination of the endoscope is not activated, light emitted from the light-emitting portion is included in the image acquired by the image-acquisition optical system, thereby making it possible to detect the relative angle of the insertion portion with respect to the through-hole. Accordingly, it is possible to prevent a reduction in detection accuracy due to halation etc. caused when illumination of the endoscope is used. On the other hand, if illumination of the endoscope is not used when the insertion portion is inserted into the through-hole of the trocar, the inside of the through-hole is usually dark; therefore, light from the light-emitting portion can be clearly detected, thus making it possible to improve the detection accuracy.

In the above-described aspect, the sign may be a window portion that can transmit external light.

By doing so, even when illumination light is not used, the relative angle between the insertion portion and the through-hole can be detected with external light shining radially inward from the window portion.

In the above-described aspect, the endoscope may be provided with: an illumination unit that emits illumination light toward a subject, at the distal end of the insertion portion; and a control unit that turns on/off the illumination unit; and the control unit may turn on the illumination unit in response to detection of the relative angle by the initial-angle detecting unit.

By doing so, the illumination unit is turned on, and an image of the inside of the trocar is acquired, thereby making it possible to confirm that the relative angle between the insertion portion and the through-hole has been detected.

According to another aspect, the present invention provides a rotation sensor fixed to a trocar that is attached to a body surface of a patient so as to penetrate the body surface and through which an insertion portion of an endoscope having an image-acquisition optical system at a distal end thereof is made to pass, the rotation sensor including: an insertion hole through which the insertion portion is made to pass; a rotation-amount detecting unit that detects an amount of rotation of the insertion portion about a longitudinal axis, the insertion portion being inserted into the insertion hole; and a sign that is disposed at a position where it can be imaged by the image-acquisition optical system when the insertion portion is inserted into the insertion hole and with which a position in a circumferential direction of the insertion hole can be identified.

According to this aspect, the rotation sensor is fixed to the trocar, which is attached so as to penetrate the body surface, and the insertion portion is inserted into the insertion hole, thereby detecting the amount of rotation of the insertion portion about the longitudinal axis, acquiring an image of the sign by using the image-acquisition optical system of the endoscope when the insertion portion is inserted into the insertion hole, and making it possible to easily detect the relative position of the insertion portion with respect to the insertion hole on the basis of the sign in the acquired image. Accordingly, it is possible to detect the absolute position of the insertion portion with respect to the insertion hole about the longitudinal axis.

According to still another aspect, the present invention provides a trocar that is attached to a body surface of a patient so as to penetrate the body surface and that has a through-hole through which an insertion portion of an endoscope having an image-acquisition optical system at a distal end thereof is made to pass, the trocar including: a rotation sensor that detects an amount of rotation of the insertion portion about a longitudinal axis, the insertion portion being located in the through-hole in a passing-through state; and a sign that is disposed at a position where it can be imaged by the image-acquisition optical system when the insertion portion is inserted into the through-hole and with which a position in a circumferential direction of the through-hole can be identified.

According to this aspect, when the trocar is attached to a body surface of a patient so as to penetrate the body surface, and the insertion portion of the endoscope is inserted into the through-hole from the distal end thereof, the rotation sensor detects the amount of rotation of the insertion portion about the longitudinal axis. Furthermore, the image-acquisition optical system, which is provided at the distal end of the insertion portion, is activated when the insertion portion is inserted into the through-hole, thereby acquiring an image including the sign, with which the position in the circumferential direction of the through-hole can be identified, and thereby making it possible to detect the relative angle between the insertion portion and the through-hole at the time of insertion on the basis of the sign in the acquired image. Specifically, the absolute position of the insertion portion of the endoscope about the longitudinal axis can be detected on the basis of the detected relative angle at the time of insertion and the amount of rotation of the insertion portion detected by the rotation sensor after insertion.

### {Advantageous Effects of Invention}

According to the present invention, an advantageous effect is afforded in that the direction of rotation can be detected with high accuracy, even when an existing endoscope is used, irrespective of the orientation of the endoscope.

### {Brief Description of Drawings}

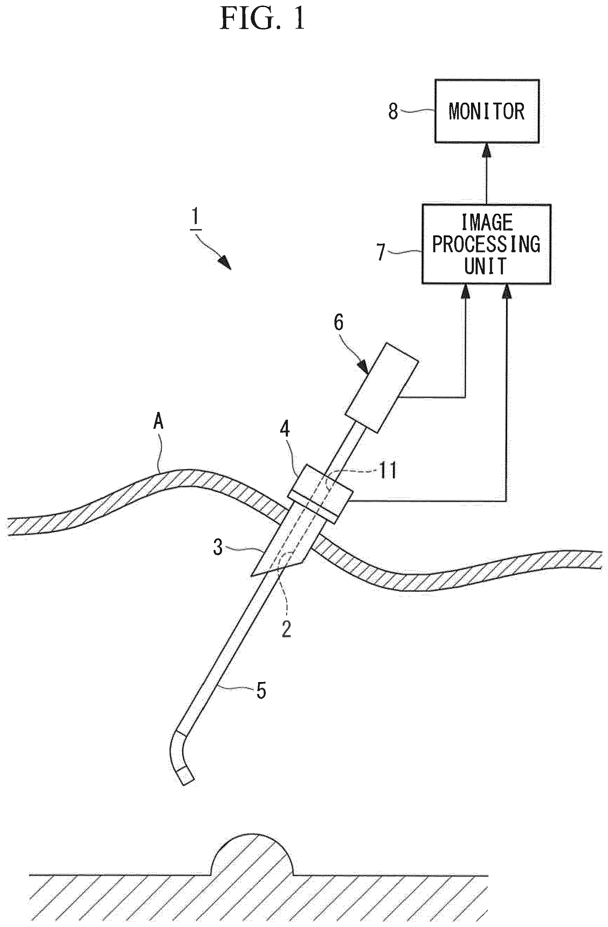

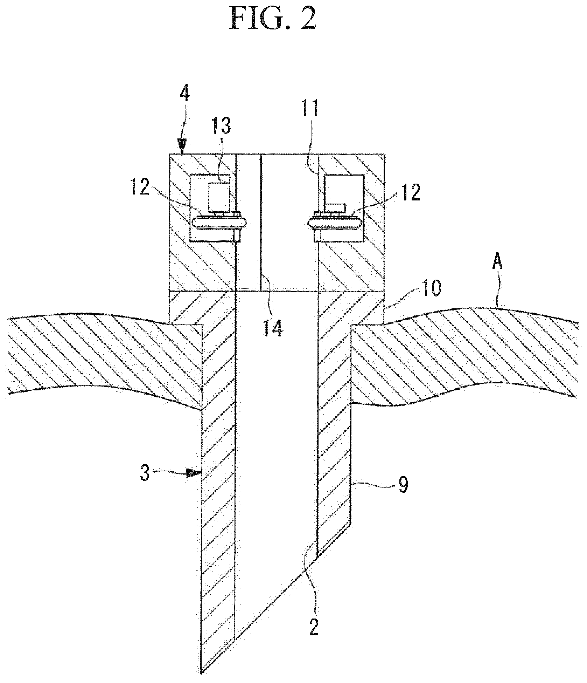

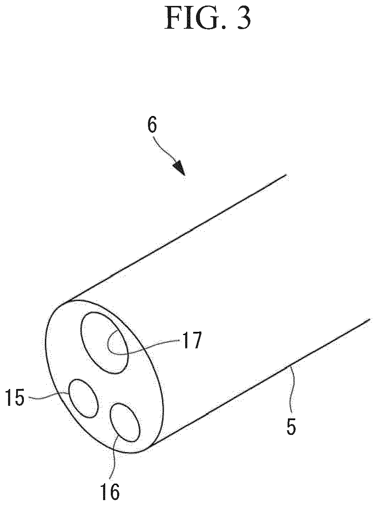

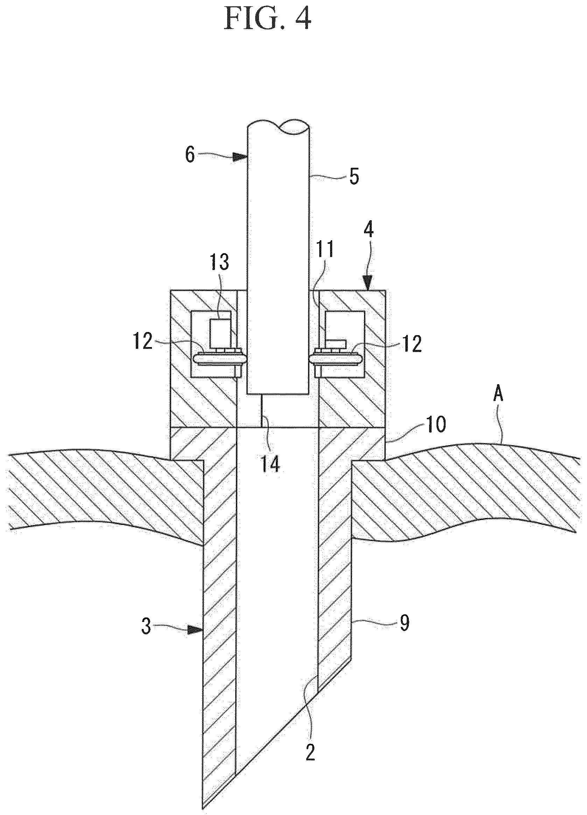

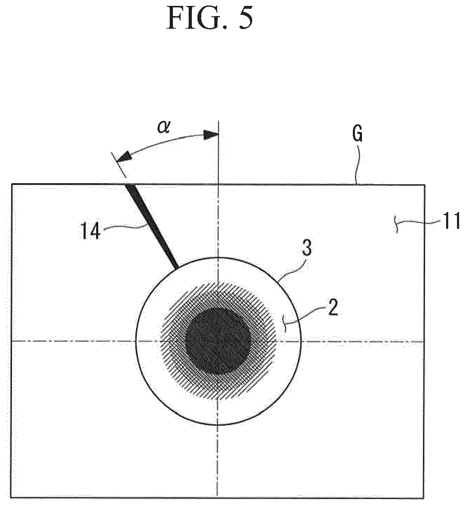

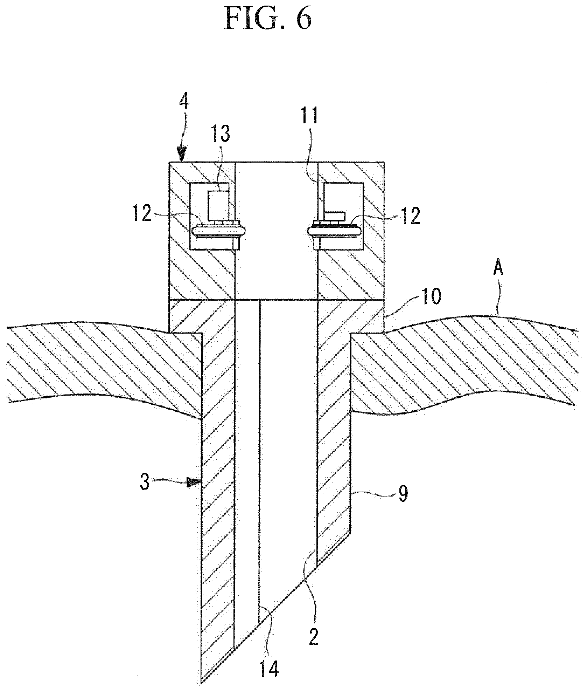

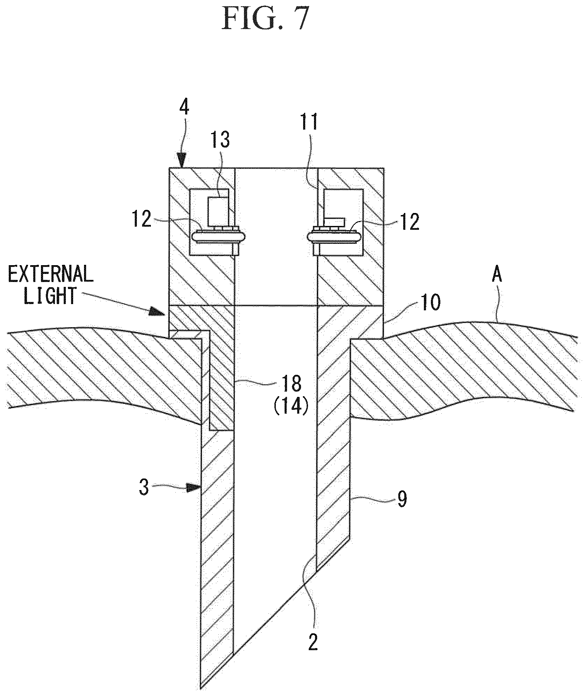

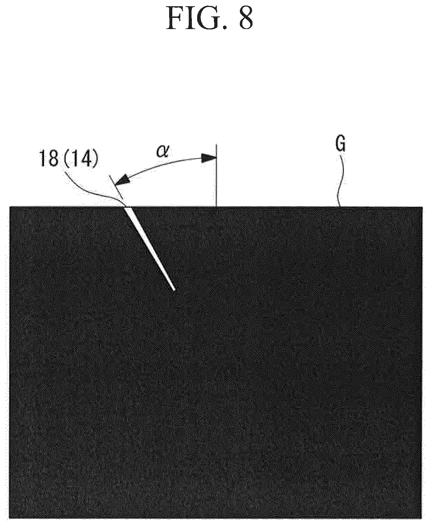

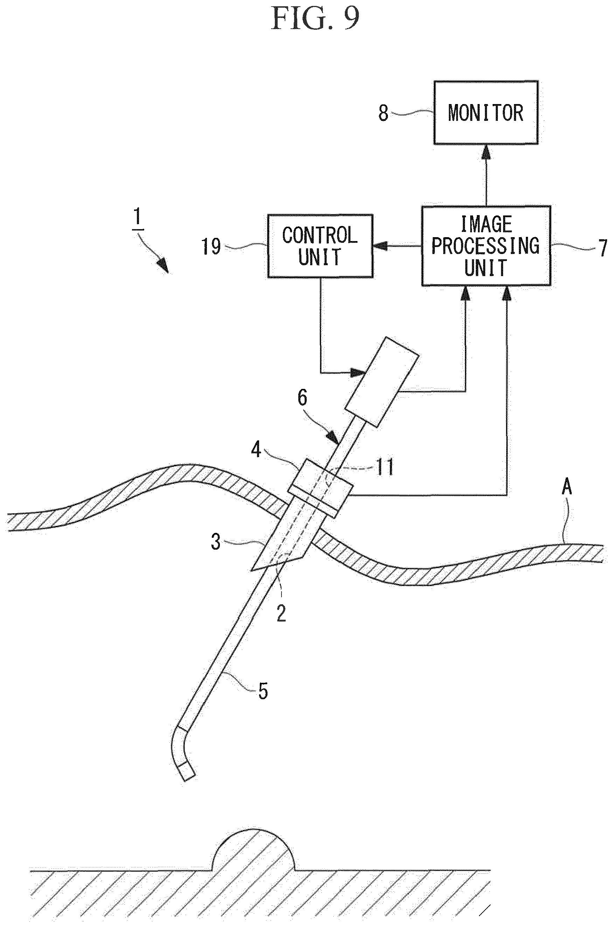

{Fig. 1} Fig. 1 is a view showing the overall configuration of an endoscope system according to one embodiment of the present invention.
{Fig. 2} Fig. 2 is a longitudinal sectional view showing a state in which a trocar and a rotation sensor that are provided in the endoscope system shown in Fig. 1 are attached so as to penetrate a body surface.
{Fig. 3} Fig. 3 is a perspective view partially showing a distal-end portion of an endoscope provided in the endoscope system shown in Fig. 1.
{Fig. 4} Fig. 4 is a longitudinal sectional view showing a state in which the distal-end portion of the endoscope is inserted into an insertion hole of the rotation sensor shown in Fig. 2.
{Fig. 5} Fig. 5 is a view showing an example image acquired by an image-acquisition optical system of the endoscope, in the state shown in Fig. 4.
{Fig. 6} Fig. 6 is a longitudinal sectional view showing a modification of the trocar shown in Fig. 2.
{Fig. 7} Fig. 7 is a longitudinal sectional view showing another modification of the trocar shown in Fig. 2.
{Fig. 8} Fig. 8 is a view showing an example image acquired by the image-acquisition optical system of the endoscope when a trocar shown in Fig. 7 is used.
{Fig. 9} Fig. 9 is a view showing the overall configuration of a modification of the endoscope system shown in Fig. 1.

### {Description of Embodiments}

An endoscope system 1 and a rotation sensor 4 according to one embodiment of the present invention will be described below with reference to the drawings.

As shown in Fig. 1, the endoscope system 1 of this embodiment is provided with: a trocar 3 that is fixed in a hole penetrating a body surface A of a patient, so as to penetrate the body surface A, and that has an insertion hole 2 for allowing the inside and the outside of the body surface A to communicate; the rotation sensor 4, which is provided on the trocar 3; an endoscope 6 that has an insertion portion 5 to be inserted into the insertion hole 2 of the trocar 3; an image processing unit (initial-angle detecting unit) 7 that processes an image G acquired by the endoscope 6; and a monitor 8 that displays the image processed by the image processing unit 7.

As shown in Fig. 2, the trocar 3 is provided with: a cylinder portion 9 that is disposed in the hole in the body surface A in a passing-through state; and a flange portion 10 that is provided at one end of the cylinder portion 9 and that is disposed outside the body in a state in which the cylinder portion 9 is mounted so as to pass through the hole in the body surface A.

The rotation sensor 4 of this embodiment is fixed to the flange portion 10 of the trocar 3 and is disposed outside the body. The rotation sensor 4 is provided with an insertion hole 11 into which the insertion portion 5 of the endoscope 6 is inserted before being inserted into the insertion hole 2 of the trocar 3, and detects the amount of rotation of the insertion portion 5, when inserted into the insertion hole 11, about the longitudinal axis. With the rotation sensor 4 fixed to the flange portion 10 of the trocar 3, the insertion hole 11 of the rotation sensor 4 and the insertion hole 2 of the trocar 3 are concentrically disposed.

For example, the rotation sensor 4 is provided with: a plurality of rollers 12 that project radially inward from the inner surface of the insertion hole 11; and an encoder (rotation-amount detecting unit) 13 that detects the amount of rotation of the rollers 12. Each of the rollers 12 has an axis line parallel to the axis of the insertion hole 11. Accordingly, when the insertion portion 5 is inserted into the insertion hole 11, the rollers 12 are brought into contact with an outer periphery of the insertion portion 5 to radially position the insertion portion 5 with respect to the insertion hole 11, and, when the insertion portion 5 is rotated about the longitudinal axis, the rollers 12 roll on the outer periphery of the insertion portion 5, and the encoder 13 detects the amount of rotation thereof.

The rotation sensor 4 of this embodiment is provided with, on the inner surface of the insertion hole 11, a single-line-shaped sign 14 that extends in a direction parallel to the axis of the insertion hole 11. The sign 14 is painted with a color different from the color of the inner surface of the insertion hole 11 or is formed by an engraving on the inner surface of the insertion hole 11.

As shown in Fig. 3, the endoscope 6 has, on a distal-end surface of the insertion portion 5, an image-acquisition optical system 15 having a forward field of view and an illumination optical system (illumination unit) 16. In the figure, reference sign 17 denotes a channel through which a treatment tool (not shown) is protruded or withdrawn.

The image processing unit 7 processes the image G acquired by the image-acquisition optical system 15, recognizes the sign 14 in the image G, and detects the direction in which the sign 14 is located, by using the coordinates held by the image-acquisition optical system 15. Accordingly, the relative angle between the insertion portion 5 and the insertion hole 11 when the insertion portion 5 of the endoscope 6 is inserted into the insertion hole 11 of the rotation sensor 4 is detected.

The operations of the thus-configured rotation sensor 4 and endoscope system 1 of this embodiment will be described below.

To observe the inside of the body cavity of a patient by using the endoscope system 1 of this embodiment, as shown in Fig. 2, the trocar 3 is inserted into a hole formed by incising the body surface A of the patient and is fixed in a passing-through state. In this state, the rotation sensor 4 has already been fixed to the flange portion 10 of the trocar 3, with the flange portion 10 disposed outside the body.

Next, the image-acquisition optical system 15 of the endoscope 6 is turned on to be in a state capable of acquiring an image G, and the insertion portion 5 is inserted into the insertion hole 11 of the rotation sensor 4 from the distal end thereof. Then, when insertion of the distal end of the insertion portion 5 into the insertion hole 11 of the rotation sensor 4 is started, the inner surface of the insertion hole 11 is imaged, and thus, the sign 14, which is provided on the inner surface, is shown in the image G, as shown in Fig. 5.

As shown in Fig. 4, when the insertion portion 5 is inserted into the insertion hole 11, the rollers 12, which are disposed in the insertion hole 11, are brought into contact with the outer periphery of the insertion portion 5 to position the insertion portion 5 in the insertion hole 11, and rotation of the insertion portion 5 about the longitudinal axis can be detected by the encoder 13.

At this point of time, the image processing unit 7 subjects the acquired image G to image recognition processing, to detect the position of the sign 14 in the image G.

For example, when the image G shown in Fig. 5 is acquired as an image at the time of insertion of the insertion portion 5, the line-shaped sign 14 inclined in one direction is shown around the center of the coordinates held by the image-acquisition optical system 15. Therefore, the image processing unit 7 detects the position of the sign 14 in the image G and detects an inclination angle α of the sign 14 around the center of the coordinates held by the image-acquisition optical system 15, as the relative angle between the insertion portion 5 and the insertion hole 11 at the time of insertion of the insertion portion 5.

Specifically, according to the rotation sensor 4 and the endoscope system 1 of this embodiment, the relative angle between the insertion portion 5 and the insertion hole 11 at the time of insertion of the insertion portion 5 of the endoscope 6 into the insertion hole 11 can be detected with the sign 14, which is provided on the inner surface of the insertion hole 11; and the amount of rotation of the insertion portion 5 about the longitudinal axis with respect to the insertion hole 11 after insertion of the insertion portion 5 into the insertion hole 11 can be detected through the operation of the rotation sensor 4. As a result, there is an advantage in that the absolute (angle) position of the insertion portion 5 about the longitudinal axis can be easily detected.

In particular, a special sensor, such as a level, for detecting the direction of gravitational force is not required to be provided in the endoscope 6; thus, it is possible to detect the direction of rotation and the amount of rotation with high accuracy even when an existing endoscope is used. Furthermore, instead of detecting the direction of gravitational force, the imaging function originally possessed by the endoscope 6 is used; therefore, there is an advantage in that the direction of rotation can be detected with high accuracy irrespective of the orientation of the endoscope 6.

According to the rotation sensor 4 and the endoscope system 1 of this embodiment, the sign 14 is provided on the inner surface of the insertion hole 11 at one place in the circumferential direction; therefore, as long as the sign 14 is shown in the acquired image G, the relative angle between the insertion portion 5 and the insertion hole 11 can be detected with high accuracy. Because the sign 14 is formed into a line shape extending in a direction parallel to the axis of the insertion hole 11, it is possible to keep detecting the sign 14 over a certain time range during the process of inserting the insertion portion 5 into the insertion hole 11.

Specifically, in order to insert the insertion portion 5 into the insertion hole 11 of the rotation sensor 4 to activate the rotation-amount detecting function of the rotation sensor 4, it is necessary to bring the rollers 12 into close contact with the outer periphery of the insertion portion 5 to accurately convert the rotation of the insertion portion 5 about the longitudinal axis into the rotations of the rollers 12. According to this embodiment, the sign 14 is not instantaneously detected but is detected over a certain time range during the process of inserting the insertion portion 5 into the insertion hole 11. Therefore, after the rotation-amount detecting function of the rotation sensor 4 is activated, for example, when the sign 14 is detected for the last time, the amount of rotation detected by the rotation sensor 4 is reset to zero, thereby making it possible to detect the absolute position with high accuracy.

Note that, in this embodiment, a description has been given of the rotation sensor 4, which has the sign 14 on the inner surface of the insertion hole 11 and which is used while being mounted on the trocar 3, and the endoscope system 1, which is provided with the same; however, as shown in Fig. 6, the sign 14 may be provided on the inner surface of the insertion hole 2 of the trocar 3. Furthermore, it is also possible to adopt a trocar 3 that is integrally provided with the rotation sensor 4 and that has the sign 14 provided on the inner surface of the insertion hole 11 or the insertion hole 2.

When the sign 14 is provided on the trocar 3, the sign 14 is detected after the insertion portion 5 passes through the rotation sensor 4; therefore, there is an advantage in that the relative angle between the insertion portion 5 and the insertion hole 2 can be detected after the rotation-amount detecting function of the rotation sensor 4 is activated, thus making it possible to more reliably detect the absolute position.

Furthermore, in this embodiment, although the sign 14 is formed of paint or an engraving, the sign 14 is not limited thereto.

For example, as shown in Fig. 7, the sign 14 may be formed of a window portion (light-emitting portion) 18 that is made of a high-light-transmittance material, such as glass, resin, or the like and that is disposed at one place in the circumferential direction of the insertion hole 2 of the trocar 3, so as to pass through from the inner surface to an outer surface of the trocar 3 disposed outside the body.

By doing so, external light is transmitted through the window portion 18 and is radiated radially inward from the inner surface of the insertion hole 2. Accordingly, the line-shaped shining sign 14 is shown in the image G acquired by the image-acquisition optical system 15 of the endoscope 6. Then, by adopting this configuration, even when the illumination optical system 16 of the endoscope 6 is not activated, the thin-line-shaped window portion 18, through which external light passes, can be detected as the sign 14.

In particular, when the insertion portion 5 is inserted, the inside of the insertion hole 2 goes black, as shown in Fig. 8; thus, the sign 14, which is formed of the line-shaped window portion 18, is shown more clearly in the image G, thus making it possible to improve the detection accuracy. Furthermore, because it is not necessary to activate the illumination optical system 16 of the endoscope 6, it is possible to prevent the occurrence of a problem in that, as in the case in which the illumination optical system 16 of the endoscope 6 is activated, illumination light having an extremely high intensity is reflected on the inner surface of the insertion hole 2 to cause halation, thus reducing the accuracy of detecting the sign 14.

Furthermore, instead of the window portion 18, the sign 14 may also be formed of an illuminant that emits light, such as an LED or organic EL, thus making it possible to obtain the same effect.

Furthermore, in this embodiment, as shown in Fig. 9, the endoscope 6 may be provided with a control unit 19 that controls the illumination optical system (illumination unit) 16. In this case, the control unit 19 turns on the illumination optical system 16 to radiate illumination light, on receiving a signal from the rotation sensor 4 and the image processing unit 7, when the rotation sensor 4 starts detecting the amount of rotation of the insertion portion 5, and the image processing unit 7 recognizes the sign 14 to detect the relative angle between the insertion portion 5 and the insertion hole 2 about the longitudinal axis at the time of insertion of the insertion portion 5 into the insertion hole 2.

By doing so, there is an advantage in that activation of the illumination optical system 16 is stopped while the relative angle at the time of insertion is being detected, thus making it possible to prevent erroneous detection of the sign 14, and in that the illumination optical system 16 is activated to acquire the image G of the inside of the insertion hole 2 of the trocar 3 or the inside of the body cavity, thus making it possible to confirm that the sign 14 is recognized with high accuracy and that the absolute position is detected.

Furthermore, in this embodiment, the line-shaped sign 14 is disposed at one place in the circumferential direction; however, the sign 14 is not limited thereto.

For example, the sign 14 may be formed into a dot-like shape, instead of the line shape, or a plurality of dots may be arranged in a direction parallel to the axis of the insertion hole 2. Furthermore, the sign 14 may be: a pattern other than the line shape or the dot shape; a letter; or a number.

Furthermore, a plurality of line-shaped signs 14 may be provided at intervals in the circumferential direction. In this case, the intervals between the lines or the widths thereof may be made different from each other. By doing so, in a case of using a side-viewing endoscope or an obliqueviewing endoscope with which the entire insertion hole 2 cannot be included in the field of view, as long as a field of view in which two or more lines can be imaged at the same time is provided, it is possible to detect the direction of the field of view in the circumferential direction.

Furthermore, the ratio of the intervals between adjacent lines or the ratio of the widths of adjacent lines may be made different from each other. By doing so, even when there is backlash between the insertion portion 5 and the insertion hole 2, or even when the observation magnification is changed, as long as a field of view in which three or more lines can be imaged at the same time is provided, it is possible to detect the direction of the field of view in the circumferential direction.

Furthermore, the colors of the signs 14 may be made different in the circumferential direction.

Furthermore, when the sign 14 is formed of light sources, the frequencies of blinking of the light sources may be made different in the circumferential direction.

### {Reference Signs List}

- 1: endoscope system
- 2: through-hole
- 3: trocar
- 4: rotation sensor
- 5: insertion portion
- 6: endoscope
- 7: image processing unit (initial-angle detecting unit)
- 11: insertion hole
- 13: encoder (rotation-amount detecting unit)
- 15: image-acquisition optical system
- 16: illumination optical system (illumination unit)
- 18: window portion (sign, light-emitting portion)
- 19: control unit
- A: body surface
- G: image

## Claims

1. An endoscope system comprising:
an endoscope in which an image-acquisition optical system is provided at a distal end of an elongated insertion portion;
a trocar that is attached to a body surface of a patient so as to penetrate the body surface and that has a through-hole through which the insertion portion is made to pass;
a rotation sensor that is provided on the trocar and that detects an amount of rotation of the insertion portion about a longitudinal axis, the insertion portion being located in the through-hole in a passing-through state;
a sign that is disposed at a position where it can be imaged by the image-acquisition optical system of the endoscope when the insertion portion is inserted into the trocar and with which a position in a circumferential direction of the through-hole can be identified; and
an initial-angle detecting unit that detects a relative angle between the insertion portion and the through-hole at the time of insertion on the basis of the sign shown in an image acquired by the image-acquisition optical system.

2. An endoscope system according to claim 1, wherein the sign is disposed at a position where it is imaged after the amount of rotation of the insertion portion can be detected by the rotation sensor.

3. An endoscope system according to claim 1 or 2, wherein the sign is provided at one place in the circumferential direction.

4. An endoscope system according to claim 3, wherein the sign is provided along a direction in which the insertion portion is inserted into the through-hole.

5. An endoscope system according to one of claims 1 to 4, wherein the sign is a light-emitting portion that can emit light radially inward.

6. An endoscope system according to claim 5, wherein the sign is a window portion that can transmit external light.

7. An endoscope system according to claim 5 or 6,
wherein the endoscope is provided with: an illumination unit that emits illumination light toward a subject, at the distal end of the insertion portion; and a control unit that turns on/off the illumination unit; and
the control unit turns on the illumination unit in response to detection of the relative angle by the initial-angle detecting unit.

8. A rotation sensor fixed to a trocar that is attached to a body surface of a patient so as to penetrate the body surface and through which an insertion portion of an endoscope having an image-acquisition optical system at a distal end thereof is made to pass, the rotation sensor comprising:
an insertion hole through which the insertion portion is made to pass;
a rotation-amount detecting unit that detects an amount of rotation of the insertion portion about a longitudinal axis, the insertion portion being inserted into the insertion hole; and
a sign that is disposed at a position where it can be imaged by the image-acquisition optical system when the insertion portion is inserted into the insertion hole and with which a position in a circumferential direction of the insertion hole can be identified.

9. A trocar that is attached to a body surface of a patient so as to penetrate the body surface and that has a through-hole through which an insertion portion of an endoscope having an image-acquisition optical system at a distal end thereof is made to pass, the trocar comprising:
a rotation sensor that detects an amount of rotation of the insertion portion about a longitudinal axis, the insertion portion being located in the through-hole in a passing-through state; and
a sign that is disposed at a position where it can be imaged by the image-acquisition optical system when the insertion portion is inserted into the through-hole and with which a position in a circumferential direction of the through-hole can be identified.
